# EUROPEAN PATENT SPECIFICATION

(11) **EP 3 488 764 B1**
(45) Date of publication and mention of the grant of the patent: **27.10.2021**
(21) Application number: 16909601.3
(22) Date of filing: 18.11.2016
(51) Int. Cl.: G01J 3/02, G01J 3/10, G01J 3/28, G01J 3/50, G01B 11/30, A61B 5/00, G01N 21/47

(54) **ELECTRONIC DEVICE FOR MEASURING AND/OR CARING SKIN CONDITIONS**
ELEKTRONISCHES GERÄT ZUM MESSEN UND/ODER PFLEGEN VON HAUTZUSTÄNDEN
DISPOSITIF ÉLECTRONIQUE POUR MESURER ET/OU SOIGNER L'ÉTAT DE LA PEAU

(30) Priority: 22.07.2016 KR 20160093178
(43) Date of publication of application: 29.05.2019
(73) Proprietor: LG Electronics Inc., Seoul 07336 (KR)
(72) Inventor: LIM, Gueisam, Seoul 06772 (KR); CHOI, Kyuhyoung, Seoul 06772 (KR); EO, Haeseok, Seoul 06772 (KR); KIM, Dongwon, Seoul 06772 (KR); JI, Eunsook, Seoul 06772 (KR); PARK, Jiyoung, Seoul 06772 (KR)
(74) Representative: Schornack, Oliver
(86) International application number: PCT/KR2016/013368
(87) International publication number: WO 2018/016686

(56) References cited:
- EP-A1- 2 753 261
- WO-A1-2006/069443
- WO-A1-2015/147535
- JP-A- 2005 034 609
- JP-A- 2011 041 706
- KR-A- 20110 008 003
- KR-B1- 101 444 940
- KR-Y1- 200 329 543
- US-A1- 2005 154 382
- US-A1- 2016 121 108

## Description

### [Technical Field]

The present disclosure relates to an electronic device. More specifically, the present disclosure relates to an electronic device capable of measuring and/or caring skin conditions.

### [Background Art]

Recently, interest in skin has been increasing. In general, dermatologic care and procedures are at a disadvantage in the high cost. Further, it is difficult to accurately measure or diagnose your skin condition, and thus side effects are often caused by using cosmetics that are not suitable for you.

The skin condition may be evaluated by various factors. Factors such as elasticity, oil, moisture, and wrinkles of the skin may be some criteria for evaluating the skin condition. You can manage your own skin if you know precise measurement indicators or history for the skin condition.

Conventional skin measuring devices and/or conventional skin care devices have a problem that their precision or accuracy is reduced when they are large-sized devices or small-sized devices for a skin clinic.

For instance, WO 2015/147535 A1 relates to a portable multifunctional skincare device having a first head and a second head. The first head includes a plurality of electrodes for outputting a micro current. The second head includes a galvanic ion electrode for generating anion and cation, a plurality of LEDs emitting a blue wavelength band light, and a camera for photographing the skin condition.

In order to support and increase functionality of the electronic device, it may be considered to improve a structural part and/or a software part of the electronic device.

### [Disclosure]

### [Technical problem]

It is an object of the present invention to provide an electronic device allowing improved speckle imaging, while maintaining a compact device.

Another object of the present disclosure is to measure appropriately, precisely or accurately a user's skin condition.

Another object of the present disclosure is to improve the quality of a measured image of the skin.

These objects are solved by the present invention as defined in independent claim 1. Preferred embodiments are defined by the dependent claims.

### [Technical Solution]

In one aspect to better understand the present disclosure, there is provided an electronic device comprising a body having an accommodation space therein; a first head extended from the body in one direction and having an accommodation space therein; a light transmission circle formed on a part of the first head; a plurality of first light emitting sources mounted on the accommodation space of at least one of the body and the first head and providing light for speckle imaging to the light transmission circle; an image sensor and a lens positioned between the light transmission circle and the plurality of first light emitting sources, spaced from the plurality of first light emitting sources, and facing the light transmission circle; a second head extended from the body in another direction; a first electrode and a second electrode positioned on an external surface of the second head; and a second light emitting source positioned adjacent to the first electrode or the second electrode and providing light to an outside of the second head.

According to another aspect of the present disclosure, the electronic device may further comprise a controller configured to analyze an image obtained by the image sensor and calculate an indicator.

According to another aspect of the present disclosure, the controller may drive at least one of the first electrode, the second electrode, and the second light emitting source according to the calculated indicator.

According to another aspect of the present disclosure, the electronic device may further comprise a third light emitting source positioned around the lens and providing light for taking an image.

According to another aspect of the present disclosure, the third light emitting source may be provided in plural, and the plurality of third light emitting sources may provide light of at least one of an ultraviolet region, a visible light region, and a near-infrared region.

According to another aspect of the present disclosure, the plurality of first light emitting sources may be laser diodes.

According to another aspect of the present disclosure, the electronic device may further comprise a housing accommodating the first light emitting sources, the image sensor, and the lens.

According to another aspect of the present disclosure, the housing may include a housing body, a housing leg, and a housing holder.

According to another aspect of the present disclosure, the housing holder may accommodate the first light emitting sources.

According to another aspect of the present disclosure, the housing body may include a housing accommodation portion accommodating the image sensor and the lens and may be positioned between the light transmission circle and the housing holder.

According to another aspect of the present disclosure, the housing leg may connect the housing body to the housing holder.

According to another aspect of the present disclosure, the housing holder may include a first housing holder and a second housing holder.

According to another aspect of the present disclosure, wherein the first housing holder and the second housing holder may be disposed asymmetrically about a virtual line connecting the housing accommodation portion and the light transmission circle.

According to another aspect of the present disclosure, the first housing holder and the second housing holder may be spaced from the housing accommodation portion by the same distance.

According to another aspect of the present disclosure, a virtual straight line connecting the first housing holder and the second housing holder may not meet a virtual straight line connecting the housing accommodation portion and the light transmission circle.

According to another aspect of the present disclosure, the first housing holder, the second housing holder, the housing accommodation portion, and the light transmission circle may be disposed corresponding to vertexes of a triangular pyramid, respectively.

According to another aspect of the present disclosure, the electronic device may further comprise a housing including a plurality of housing holders accommodating the plurality of first light emitting sources; a housing body including a housing accommodation portion accommodating the image sensor, the housing body being positioned between the light transmission circle and the plurality of housing holders; and a plurality of housing legs connecting the housing body to the plurality of housing holders.

According to another aspect of the present disclosure, the electronic device may further comprise a rear case coupled to a rear of the housing and accommodating the first light emitting sources.

According to another aspect of the present disclosure, the rear case may be coupled to the rear of the housing, may contact the first light emitting sources, and may include a metal.

According to another aspect of the present disclosure, the rear case may be connected to the first light emitting sources and may receive hear from the first light emitting sources to discharge the heat.

According to another aspect of the present disclosure, at least one of the housing and the rear case may include a light absorbing material on an external surface.

According to another aspect of the present disclosure, the first electrode and the second electrode each may have an electric potential. The first electrode may have a first electric potential, and the second electrode may have a second electric potential different from the first electric potential.

According to another aspect of the present disclosure, the electronic device may further comprise a third electrode positioned adjacent to at least one of the first electrode and the second electrode and providing light of an infrared region.

According to another aspect of the present disclosure, the electronic device may further comprise a wireless communication unit mounted on the body, and the wireless communication unit may transmit a skin indicator of a user measured by the electronic device to another electronic device.

### [Advantageous Effects]

Effects of an electronic device according to the present disclosure are described as follows.

According to at least one aspect of the present disclosure, the present disclosure can measure appropriately, precisely or accurately a user's skin condition.

According to at least one aspect of the present disclosure, the present disclosure can perform speckle imaging by providing laser light.

According to at least one aspect of the present disclosure, the present disclosure can improve the quality of a measured image of the skin.

According to at least one aspect of the present disclosure, the present disclosure can measure a user's skin condition, store or analyze information on the user's skin condition, and provide a skin care suitable for the information.

According to at least one aspect of the present disclosure, the present disclosure can share information about a skin measurement with another electronic device.

Further scope of applicability of the present disclosure will become apparent from the detailed description given hereinafter. However, it should be understood that the detailed description and specific examples, while indicating exemplary embodiments of the disclosure, are given by illustration only. The scope of the present invention is defined by the claims.

### [Description of Drawings]

FIG. 1 illustrates an example of an electronic device according to an embodiment of the disclosure.
FIG. 2 is a figure of an electronic device of FIG. 1 viewed from a side of a first head.
FIG. 3 is a figure of an electronic device taken along A-A' of FIG. 2.
FIG. 4 illustrates examples of an optical element according to an embodiment of the disclosure.
FIGS. 5 and 6 illustrate examples of an image measurement according to an embodiment of the disclosure.
FIGS. 7 to 10 illustrate examples of an electronic device according to an embodiment of the disclosure.
FIG. 11 illustrates a part of an electronic device according to an embodiment of the disclosure.
FIG. 12 illustrates examples of a measurement image according to an embodiment of the disclosure.
FIG. 13 illustrates an example of order of skin measurement according to an embodiment of the disclosure.
FIGS. 14 and 15 illustrate examples of a result of measuring skin conditions according to an embodiment of the disclosure.
FIG. 16 illustrates other examples of a result of measuring skin conditions according to an embodiment of the disclosure.
FIG. 17 is a figure of an electronic device of FIG. 1 viewed from a side of a second head.
FIG. 18 is a cross-sectional view of an electronic device taken along B-B' of FIG. 17.
FIG. 19 illustrates an example of a front surface of a second head according to an embodiment of the disclosure.
FIG. 20 illustrates another example of a front surface of a second head according to an embodiment of the disclosure.
FIGS. 21 to 23 illustrate implementations of a skin care performed by an electronic device according to an embodiment of the disclosure.
FIGS. 24 to 27 illustrate examples of a skin measurement and a skin care according to an embodiment of the disclosure.
FIGS. 28 to 30 illustrate an example of another electronic device related to an electronic device according to an embodiment of the disclosure.

### [Mode for Invention]

Reference will now be made in detail to embodiments of the disclosure, examples of which are illustrated in the accompanying drawings. Wherever possible, the same reference numbers will be used throughout the drawings to refer to the same or like parts. In general, a suffix such as "module" and "unit" may be used to refer to elements or components. Use of such a suffix herein is merely intended to facilitate description of the specification, and the suffix itself is not intended to give any special meaning or function. It will be noted that a detailed description of known arts will be omitted if it is determined that the detailed description of the known arts can obscure the embodiments of the disclosure. The accompanying drawings are used to help easily understand various technical features, and it should be understood that the embodiments presented herein are not limited by the accompanying drawings. As such, the present disclosure should be construed to extend to any alterations, equivalents and substitutes in addition to those which are particularly set out in the accompanying drawings.

Terms including ordinals such as first, second, etc. may be used to describe various components, but the components are not limited by the terms. The terms are used only for the purpose of distinguishing one component from other components.

When an arbitrary component is described as "being connected to" or "being coupled to" other component, this should be understood to mean that still another component(s) may exist between them, although the arbitrary component may be directly connected or directly coupled to the other component. On the other hand, when an arbitrary component is described as "being directly connected to" or "being directly coupled to" another component, this should be understood to mean that no component exists between them.

A singular expression includes a plural expression as long as it does not have an apparently different meaning in context.

In the present application, the terms of "include" or "have", etc. should be understood to be intended to designate that illustrated features, numbers, steps, operations, components, parts or combinations thereof exist and not to preclude the existence of one or more different features, numbers, steps, operations, components, parts or combinations thereof, or the possibility of the addition thereof.

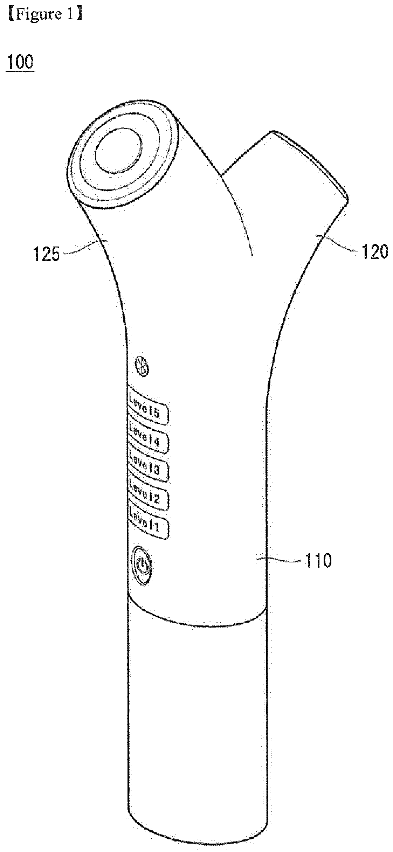

FIG. 1 illustrates an example of an electronic device according to an embodiment of the disclosure (maintaining the same as the brief description of the drawings: omitted below). A body 110 may have an extended shape. Further, the body 110 Provides an internal space.

A first head 120 is extended from the body 110 in one direction. The first head 120 may be formed in one body with the body 110, or may be formed separately from the body 110 and then coupled to the body 110. The first head 120 has an accommodation space therein.

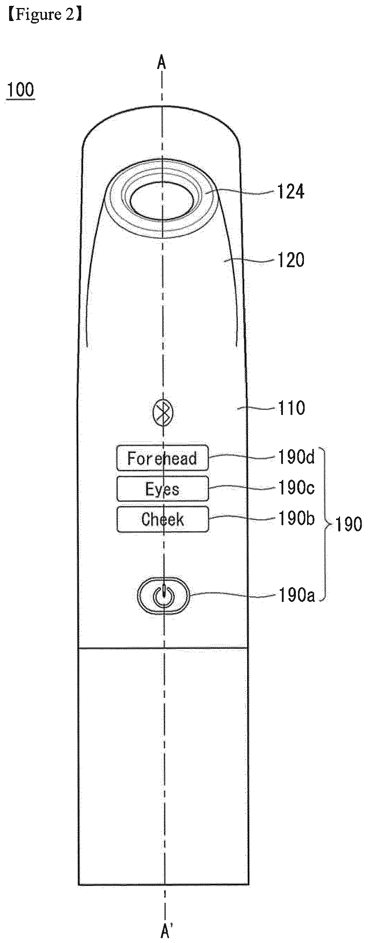

A second head 125 is extended from the body 110 in another direction. The extending direction of the second head 125 from the body 110 is be different from the extending direction of the first head 120 from the body 110. The second head 125 may be formed in one body with the body 110. The second head 125 may be formed separately from the body 110 and then coupled to the body 110. The second head 125 may have an accommodation space therein. FIG. 2 is a figure of the electronic device of FIG. 1 viewed from the side of the first head. A contact portion 124 may form a part of an upper surface of the first head 120. The contact portion 124 may be coupled to one side of the first head 120. The contact portion 124 may be positioned on a front surface of the first head 120.

The contact portion 124 may be directed toward objects other than an electronic device 100. For example, the contact portion 124 may be directed toward a user's skin. For example, the contact portion 124 may contact the user's skin. An operation unit 190 may receive an input from a user. The operation unit 190 may include at least one of a button, an SMT dome, and a touch panel. The touch panel may obtain a touch input. The touch panel may display a screen. The operation unit 190 may include a first operation unit 190a, a second operation unit 190b, a third operation unit 190c, and a fourth operation unit 190d.

The first operation unit 190a may obtain an input for power supply of at least some of the components included in the electronic device 100. For example, the first operation unit 190a may obtain an input for power supply of a component connected to the first head 120. For example, the input for power supply may mean turning on/off the power. For example, if the user presses the first operation unit 190a, the skin measurement may be started. In addition, if the user presses the first operation unit 190a while the skin measurement is continuing, the skin measurement may be ended.

The second to fourth operation units 190b, 190c and 190d may be related to the selection of functions of the electronic device 100. The second to fourth operation units 190b, 190c and 190d may be related to information sent to the electronic device 100. For example, information corresponding to an input obtained by the second operation unit 190b may be different from information corresponding to an input obtained by the third and/or fourth operation units 190c and 190d.

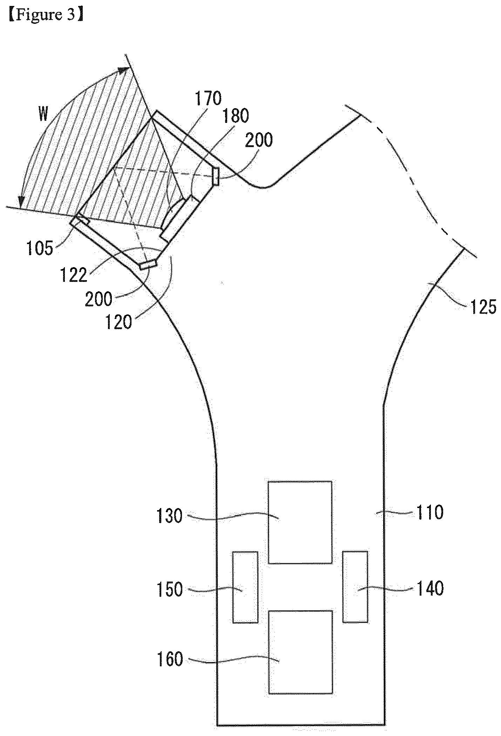

FIG. 3 is a cross-sectional view of the electronic device taken along A-A' of FIG. 2. The first head 120 is extended from the body 110 in one direction. The first head 120 may form a recessed area 122. The recessed area 122 may be formed from the front surface of the first head 120 toward the inside of the first head 120.

The second head 125 is extended from the body 110 in another direction. FIG. 3 illustrates a part of the second head 125. Configuration disposed at the second head 125 is omitted in FIG. 3.

A main circuit board 130 may be mounted in the internal space of the body 110. The main circuit board 130 may be mounted with electronic elements required for the electronic device 100. Alternatively, the main circuit board 130 may control electronic elements included in the electronic device 100.

A light control unit 140 may be mounted in the internal space of the body 110. The light control unit 140 may control a light emitting source 200. For example, the light control unit 140 may include a relay that can adjust a current supplied to the light emitting source 200.

A wireless communication unit 150 may enable the electronic device 100 to perform wireless communication with another electronic device. For example, the wireless communication unit 150 may be a module that enables communication such as Wi-Fi, BT, and NFC.

A power supply unit 160 may supply power to the electronic elements included in the electronic device 100. The power supply unit 160 may be a secondary cell. For example, the power supply unit 160 may be a Li-ion battery.

A lens 170 is provided in the recessed area 122 of the body 110. The lens 170 may be a wide-angle (w) lens. A sensor 180 receives external light of the electronic device through the lens 170 and may convert the light into an image. For example, the sensor 180 may be a CMOS, a CCD, etc. For example, the sensor 180 is an image sensor 180.

The light emitting source 200 is disposed on at least one of the first head 120 and the body 110. For example, the light emitting source 200 is provided in the recessed area 122 of the first head 120. For example, the light emitting source 200 may be mounted in the recessed area 122 so that it is positioned around the lens 170. The light emitting source 200 includes a plurality of light emitting sources.

A reference chart 105 may be positioned at one side of the recessed area 122 of the first head 120. For example, the reference chart 105 may be RGB or Gray scale. The reference chart 105 may provide criteria of image information obtained through the lens 170 and the sensor 180. For example, the criteria of the image information may be a white balance, a color temperature, and the like.

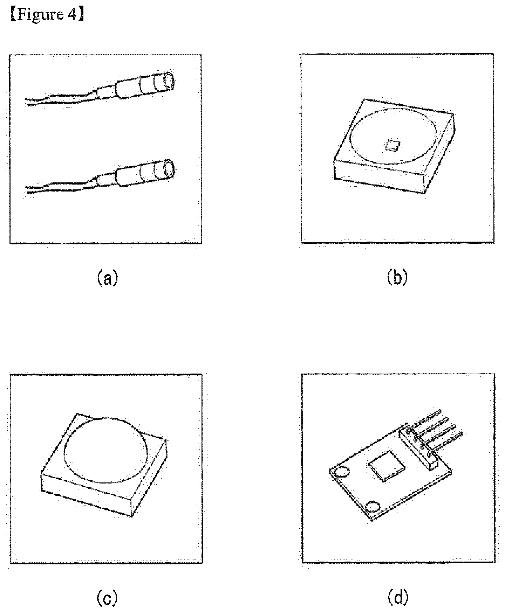

FIG. 4 illustrates a light emitting source according to several embodiments of the disclosure. FIG. 4 illustrates a laser diode in (a), a near-infrared LED in (b), an ultraviolet LED in (c), and a visible LED in (d), as an example of the light emitting source.

Structures and components identical or equivalent to those described above are designated with the same reference numerals, and a further description is omitted. In the present specification, speckle imaging may mean irradiating light of different wavelengths onto an object and measuring a state of a surface of the object or the inside close to the surface of the object using overlap or interference between the light of the different wavelengths.

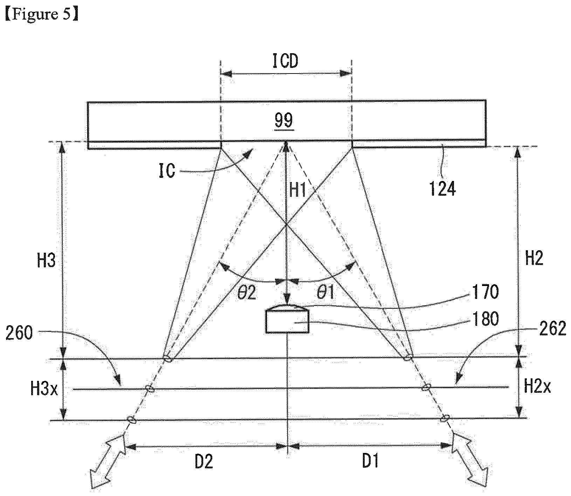

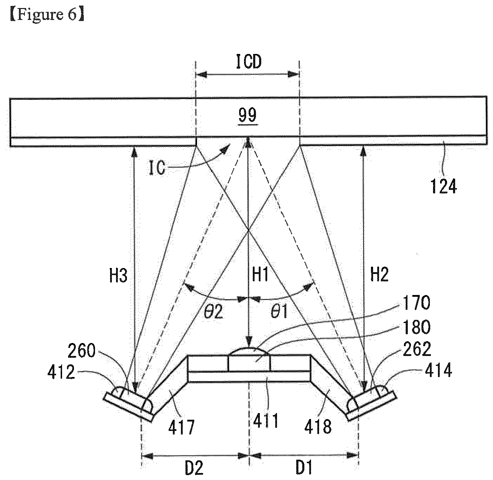

FIGS. 5 and 6 illustrate examples of an image measurement according to an embodiment of the disclosure.

Referring to FIG. 5, the first head 120 has a light transmission circle IC. The light transmission circle IC is formed on one surface of the first head 120. The light transmission circle IC may be formed adjacent to the contact portion 124. The light transmission circle IC and the contact portion 124 may form one surface or the upper surface of the first head 120.

The image sensor 180 and/or the lens 170 are directed toward the light transmission circle IC. That is, the lens 170 and the image sensor 180 may photograph an object 99 that contacts or faces the light transmission circle IC. For example, the lens 170 may be a macro lens.

A plurality of first light emitting sources 260 and 262 provides light for the speckle imaging. The plurality of first light emitting sources 260 and 262 may provide light of a region band (including an ultraviolet region band and a near-infrared region band) between ultraviolet light and near-infrared light. For example, one of the plurality of first light emitting sources 260 and 262 may have one wavelength among 450 nm, 530 nm, and 650 nm.

An axis on which the lens 170 receives light is defined as an optical axis, and an axis on which the first light emitting sources 260 and 262 irradiate light is defined as an irradiation axis. The irradiation axis has a predetermined angle with respect to the optical axis, so that light irradiated by the first light emitting sources 260 and 262 is reflected from the object 99 and measures a state of the surface (which may include not only an outer surface of the object 99 but also some depth of the object 99) of the object 99. For example, an angle θ1 at which the first light emitting source 262 irradiates light onto the object 99 is about 30 to 40 degrees. Hence, the speckle imaging can be performed effectively. An angle θ2 at which the first light emitting source 260 irradiates light onto the object 99 is about 30 to 40 degrees. Hence, diffuse reflection of the object 99 can be effectively reduced or suppressed.

In terms of the distance, for example, a distance D2 between the lens 170 and the first light emitting source 260 may be 13 to 15 mm, a distance D1 between the lens 170 and the first light emitting source 262 may be 10 mm, a distance H3 between the first light emitting source 260 and the object 99 or the light transmission circle IC may be 17 to 22 mm, a distance H2 between the first light emitting source 262 and the object 99 or the light transmission circle IC may be 20 to 25 mm, and a distance H1 between the lens 170 and the object 99 or the light transmission circle IC may be 25 to 30 mm.

The first light emitting sources 260 and 262 may be positioned to be spaced from the lens 170. The first light emitting sources 260 and 262 may be positioned around the lens 170. The first light emitting sources 260 and 262 are positioned in the rear of the image sensor 180 or the lens 170. The first light emitting sources 260 and 262 include a plurality of light emitting elements 260 and 262. The plurality of light emitting elements 260 and 262 may include a first light emitting element 260 and a second light emitting element 262.

The image sensor 180 is positioned at a predetermined distance H1 from the light transmission circle IC. The image sensor 180 is positioned on the optical axis. The light transmission circle IC may have a diameter ICD of 10 to 12 mm, for example. In this instance, the image sensor 180 and/or the lens 170 may have an angle of view entirely covering the light transmission circle IC. For example, the distance H1 of the image sensor 180 from the light transmission circle IC may be 25 to 30 mm, and the lens 170 may be a macro lens.

The first light emitting element 260 may be positioned on one side of the image sensor 180. The second light emitting element 262 may be positioned on the other side of the image sensor 180. The first light emitting element 260 and the second light emitting element 262 may be positioned on both sides of the image sensor 180. The first light emitting element 260 and the second light emitting element 262 may be disposed asymmetrically about the optical axis. In other words, when an optical axis formed by the image sensor 180 and two irradiation axes formed by the first and second light emitting elements meet at a single point, the optical axis and the two irradiation axes may not be positioned on one plane.

The first light emitting element 260 may be positioned on the left or right side of the image sensor 180 to be spaced from the image sensor 180. The distance D2 may be 13 to 15 mm. The first light emitting element 260 forms an inclined irradiation axis while maintaining a predetermined angle θ2 from an optical axis of the image sensor 180. The angle θ2 formed by the irradiation axis from the optical axis may be 25 to 35 degrees. For example, the first light emitting element 260 may be a laser diode (LD) that provides light with a wavelength of 450 nm.

The second light emitting element 262 may be positioned on the left or right side of the image sensor 180 to be spaced from the image sensor 180. The distance D1 may be 10 mm. The second light emitting element 262 forms an inclined irradiation axis while maintaining a predetermined angle θ1 from the optical axis of the image sensor 180. The angle θ1 formed by the irradiation axis from the optical axis may be 25 to 35 degrees. For example, the second light emitting element 262 may be a laser diode that provides light with a wavelength of 650 nm.

The first light emitting element 260 is positioned in the rear of the image sensor 180. That is, the first light emitting element 260 may be positioned in the left rear or the right rear of the image sensor 180. A distance H3x at which the first light emitting element 260 is spaced rearward from the image sensor 180 may correspond to a distance at which light provided by the first light emitting element 260 maintains a constant output at the light transmission circle IC. For example, light provided by the first light emitting element 260 may maintain an output of 1 mW at the light transmission circle IC.

The second light emitting element 262 is positioned in the rear of the image sensor 180. That is, the second light emitting element 262 may be positioned in the left rear or the right rear of the image sensor 180. A distance H2x at which the second light emitting element 262 is spaced rearward from the image sensor 180 may correspond to a distance at which light provided by the second light emitting element 262 maintains a constant output at the light transmission circle IC. For example, light provided by the second light emitting element 262 may maintain an output of 1 mW at the light transmission circle IC.

As another example, the first light emitting element 260 and the second light emitting element 262 may be positioned to be spaced rearward from the image sensor 180, so that light provided by the first light emitting element 260 and the second light emitting element 262 is combined on the light transmission circle IC to form an output of 1 mW.

Referring to FIG. 6, for example, the first light emitting sources 260 and 262 may be an LD that provides light with a wavelength of 405 nm or 650 nm. When the first light emitting sources 260 and 262 are the LD, light provided by the first light emitting sources 260 and 262 may have linearity. This means that a radiation angle of light is narrow.

If the first light emitting sources 260 and 262 provide light with a narrow radiation angle, the light may not be provided for the whole of the light transmission circle IC. Thus, at least a portion of the first light emitting sources 260 and 262 may be covered with the lens, in order to provide light for the whole of the light transmission circle IC. The lens connected to the first light emitting sources 260 and 262 may extend a radiation angle of light provided by the first light emitting sources 260 and 262.

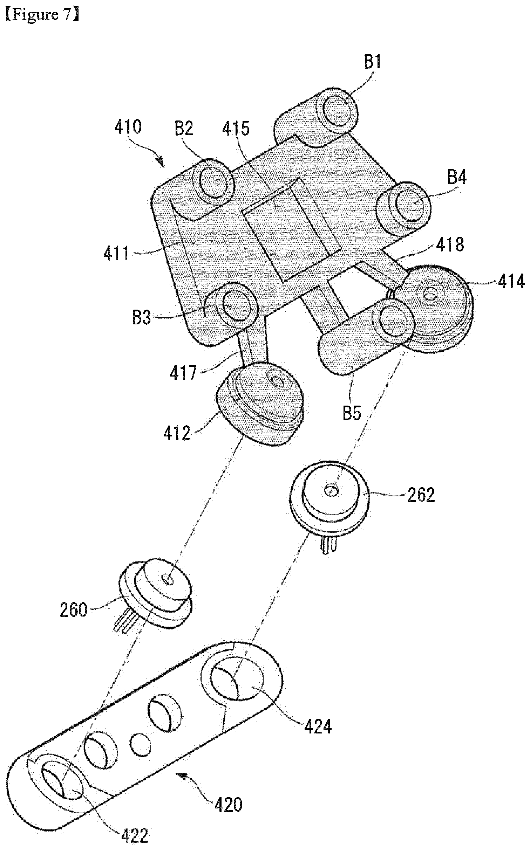

A housing 410 (shown in FIG. 7) may accommodate the lens 170 and the image sensor 180. The housing 410 may accommodate the first light emitting sources 260 and 262. The housing 410 (shown in FIG. 7) may be positioned on at least one of the body 110 (shown in FIG. 1) and the first head 120 (shown in FIG. 1). The housing 410 may maintain the relative disposition between the lens 170 and the image sensor 180 and the contact portion 124. The housing 410 may maintain the relative disposition between the lens 170 and the image sensor 180 and the first light emitting sources 260 and 262. For example, the image sensor 180 and the lens 170 may be positioned between the light transmission circle IC and the first light emitting sources 260 and 262.

The lens 170 and the image sensor 180 may be accommodated in a housing body 411. The housing body 411 may be directed toward the light transmission circle IC. The housing body 411 may be positioned on the optical axis. The housing body 411 may be spaced from the light transmission circle IC at a predetermined distance H1. For example, the housing body 411 may include a circuit board.

The image sensor 180 may be mounted on the housing body 411. The lens 170 may be mounted on the housing body 411 in alignment with the image sensor 180.

Housing holders 412 and 414 may accommodate the first light emitting sources 260 and 262. The first light emitting sources 260 and 262 may be mounted on the housing holders 412 and 414. The first light emitting sources 260 and 262 may be connected or fixed to the housing holders 412 and 414. The housing holders 412 and 414 may be provided in plural. For example, the housing holders 412 and 414 may include a first housing holder 412 and a second housing holder 414. For example, the first housing holder 412 may accommodate the first light emitting source 260. For example, the second housing holder 414 may accommodate the first light emitting source 262. The housing holders 412 and 414 may form holes through which light can pass.

The housing holders 412 and 414 may be positioned in the rear of the image sensor 180 or in the rear of the housing body 411. The housing holders 412 and 414 may be directed toward the light transmission circle IC. For example, the second housing holder 414 may form a predetermined angle θ1 with respect to the optical axis. For example, the first housing holder 412 may form a predetermined angle θ2 with respect to the optical axis. For example, an angle formed by the second housing holder 414 with respect to the light transmission circle IC may correspond to an irradiation angle formed by the first light emitting source 262. For example, an angle formed by the first housing holder 412 with respect to the light transmission circle IC may correspond to an irradiation angle formed by the first light emitting source 260.

Housing legs 417 and 418 may connect the housing holders 412 and 414 to the housing body 411. The housing legs 417 and 418 may fix the housing holders 412 and 414 to the housing body 411. The housing legs 417 and 418 may be provided in plural. For example, the housing legs 417 and 418 may include a first housing leg 417 and a second housing leg 418. For example, the first housing leg 417 may connect the first housing holder 412 to the housing body 411. For example, the second housing leg 418 may connect the second housing holder 414 to the housing body 411.

The housing legs 417 and 418 may be extended from one side of the housing holders 412 and 414. The housing legs 417 and 418 may be extended in a direction away from the lens 170 and the image sensor 180. For example, the housing legs 417 and 418 may be extended to the rear and the side of the lens 170 and the image sensor 180.

FIGS. 7 to 10 illustrate a configuration relationship of an electronic device according to an embodiment of the disclosure.

Referring to FIG. 7, the housing 410 may include the housing body 411, the housing leg 417, and the housing holders 412 and 414.

At least a portion of the housing 410 may include a material for light absorption (i.e., light absorbing material). At least a portion of an external surface of the housing 410 may include a material for light absorption.

The light absorbing material may be a material that absorbs light. For example, the light absorbing material may absorb light of a region band of at least one of visible light, infrared light, and ultraviolet light. The light absorbing material may be applied to at least a portion of the external surface of the housing 410. The light absorbing material may include a material of a size corresponding to a wavelength of electromagnetic waves to be absorbed. For example, the light absorbing material may be a material having a natural frequency corresponding to the wavelength of the absorbed electromagnetic waves. For example, the light absorbing material may have a black color. In this instance, the external surface of the housing 410 coated with the light absorbing material may have a rough surface. The external surface of the housing 410 may have a smooth surface depending on a kind of light absorbing material.

The housing body 411 may include a housing accommodation portion 415 on its one side. The housing accommodation portion 415 may be formed by depressing a portion or one surface of the housing body 411. For example, the housing accommodation portion 415 may be formed by depressing a central area of the front surface of the housing body 411. The housing accommodation portion 415 may accommodate the lens 170 and the image sensor 180.

The housing body 411 may include bosses B1, B2, B3, B4 and B5 on its one side. The bosses B1, B2, B3, B4 and B5 may be provided in plural. For example, the bosses B1, B2, B3, B4 and B5 may include a first boss B1, a second boss B2, a third boss B3, a fourth boss B4, and a fifth boss B5.

The bosses B1, B2, B3, B4 and B5 may be formed in a shape protruding from one side of the housing body 411. The bosses B1, B2, B3, B4 and B5 may be extended from one side of the housing body 411 toward the light transmission circle IC. At least a part of the plurality of bosses B1, B2, B3, B4 and B5 may be formed in a shape surrounding the housing accommodation portion 415. The plurality of bosses B1, B2, B3, B4 and B5 may have the shape surrounding the housing accommodation portion 415, and thus can maintain the stable fixing with respect to the first head 120 (shown in FIG. 1) or the body 110 (shown in FIG. 1) of the housing 410.

The housing leg 417 may be connected to one side of the housing body 411. The housing legs 417 may include a plurality of housing legs. For example, the housing leg 417 may include a first housing leg 417 and a second housing leg 418. For example, the first housing leg 417 may connect the first housing holder 412 to the housing body 411. For example, the second housing leg 418 may connect the second housing holder 414 to the housing body 411.

The first housing holder 412 may be connected to the first housing leg 417. The second housing holder 414 may be connected to the second housing leg 418. The first housing holder 412 and the second housing holder 414 may be spaced from the housing accommodation portion 415 by the same distance. The first housing holder 412 and the second housing holder 414 may be symmetric about an axis connecting the housing accommodation portion 415 and the light transmission circle IC (shown in FIG. 6). The axis connecting the housing accommodation portion 415 and the light transmission circle IC (shown in FIG. 6) may mean the optical axis shown in FIG. 6.

On the other hand, the first housing holder 412 and the second housing holder 414 may be asymmetric about an axis connecting the housing accommodation portion 415 and the light transmission circle IC (shown in FIG. 6). For example, the first housing holder 412 and the second housing holder 414 may have different distances from the axis. In this case, it may be easy to dispose the housing holders 412 and 414 correspondingly to the design of the electronic device according to an embodiment of the disclosure. In embodiments, the axis connecting the housing accommodation portion 415 and the light transmission circle IC (shown in FIG. 6) may be a virtual line. The axis connecting the housing accommodation portion 415 and the light transmission circle IC (shown in FIG. 6) may mean a virtual line connecting a center of the housing accommodation portion 415 and a center of the light transmission circle IC (shown in FIG. 6). Alternatively, a virtual straight line connecting the first housing holder 412 and the second housing holder 414 may not meet a virtual straight line connecting the housing accommodation portion 415 and the light transmission circle IC (shown in FIG. 6).

The first housing holder 412 and the second housing holder 414 may lean in a direction perpendicular to the axis connecting the housing accommodation portion 415 and the light transmission circle IC (shown in FIG. 6). In other words, the first housing holder 412, the second housing holder 414, the housing accommodation portion 415, and the light transmission circle IC (shown in FIG. 6) may be disposed corresponding to vertexes of a triangular pyramid, respectively. In this case, the space consumption of the housing can be reduced.

A rear case 420 may be connected to a part or all of the housing holders 412 and 414. The rear case 420 may be connected to the rear of the housing holders 412 and 414. The rear case 420 may accommodate the first light emitting sources 260 and 262. The rear case 420 may be connected to the first light emitting sources 260 and 262. The rear case 420 may include seating portions 422 and 424.

The rear case 420 may receive heat from the first light emitting sources 260 and 262. The rear case 420 may discharge heat from the first light emitting sources 260 and 262 to the outside. The rear case 420 may have thermal conductivity. The thermal conductivity of the rear case 420 may be considerably high. The rear case 420 may include a metal. For example, the rear case 420 may include at least one of copper, aluminum, silver, gold, iron/steel, and zinc.

The seating portions 422 and 424 may be provided in plural. For example, the seating portions 422 and 424 may include a first seating portion 422 and a second seating portion 424. The first seating portion 422 may correspond to the first housing holder 412. The second seating portion 424 may correspond to the second housing holder 414. The first seating portion 422, the first light emitting source 260, and the first housing holder 412 may be sequentially disposed. The second seating portion 424, the first light emitting source 262, and the second housing holder 414 may be sequentially disposed.

The first light emitting source 260 may be placed on the first seating portion 422. The first housing holder 412 may cover the first light emitting source 260. The first light emitting source 260 may be positioned between the first seating portion 422 and the first housing holder 412. The first seating portion 422 and the first housing holder 412 may cover the first light emitting source 260.

The first light emitting source 262 may be placed on the second seating portion 424. The second housing holder 414 may cover the first light emitting source 262. The first light emitting source 262 may be positioned between the second seating portion 424 and the second housing holder 414. The second seating portion 424 and the second housing holder 414 may cover the first light emitting source 262.

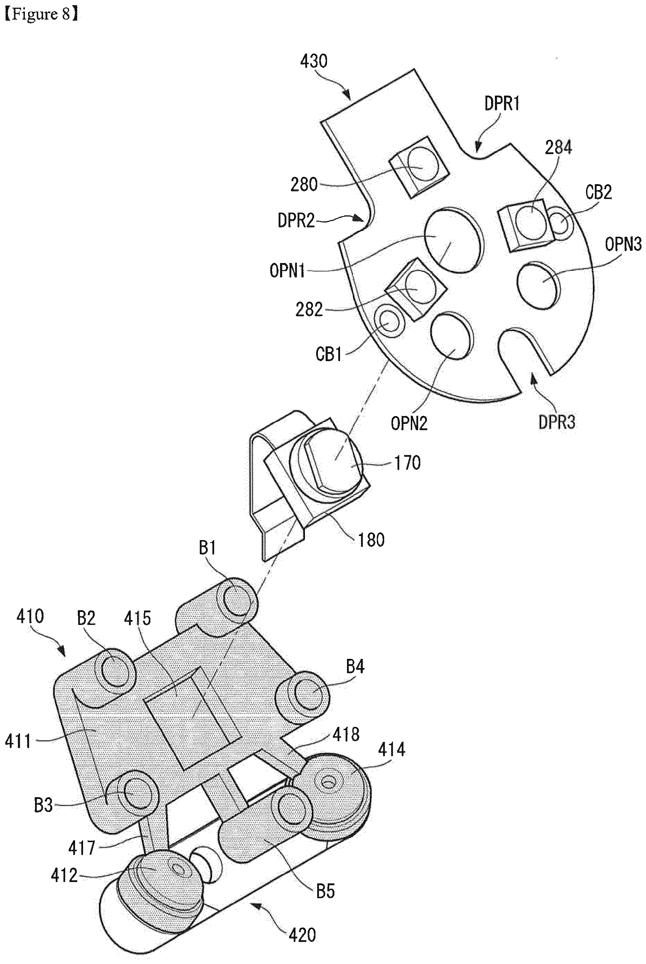

Referring to FIG. 8, a deck 430 may have a plate shape. The deck 430 may form openings OPN1, OPN2 and OPN3. For example, the openings OPN1, OPN2 and OPN3 may include a first opening OPN1, a second opening OPN2, and a third opening OPN3. The openings OPN1, OPN2 and OPN3 may pass light. For example, the openings OPN1, OPN2 and OPN3 may have a hole shape. The deck 430 may form recessed areas DPR1, DPR2 and DPR3 of a bay shape that are depressed inward. For example, the recessed areas DPR1, DPR2 and DPR3 may include a first recessed area DPR1, a second recessed area DPR2, and a third recessed area DPR3. The deck 430 may form deck coupling portions CB1 and CB2 coupled to the housing 410. For example, the deck coupling portions CB1 and CB2 may include a first deck coupling portion CB1 and a second deck coupling portion CB2.

The deck 430 may be connected to one side of the housing 410. The first deck coupling portion CB1 may be connected and fixed to the third boss B3. The second deck coupling portion CB2 may be connected and fixed to the fourth boss B4. The first recessed area DPR1 may partially accommodate the first boss B1. The second recessed area DPR2 may partially accommodate the second boss B2. The third recessed area DPR3 may partially accommodate the fifth boss B5. The deck 430 can maintain a stable coupling state, a stable coupling structure, or a stable posture by the bosses B1, B2 and B5 respectively corresponding to the first to third recessed areas DPR1, DPR2 and DPR3.

The deck 430 may be positioned in front of the housing 410. The first opening OPN1 of the deck 430 may correspond to the housing accommodation portion 415 of the housing 410. The first opening OPN1 of the deck 430 may pass or accommodate the lens 170. The second opening OPN2 may pass light emitted from the first housing holder 412. The light emitted from the first housing holder 412 may be provided by the first light emitting source 260. The third opening OPN3 may pass light emitted from the second housing holder 414. The light emitted from the second housing holder 414 may be provided by the first light emitting source 262.

Third light emitting sources 280, 282 and 284 may be mounted on the deck 430. The third light emitting sources 280, 282 and 284 may be disposed in front of the deck 430. The third light emitting sources 280, 282 and 284 may provide light toward the front of the deck 430. The front of the deck 430 may be a direction from the inside of the first body 120 toward the light transmission circle IC.

The third light emitting sources 280, 282 and 284 provide light for obtaining an image. For example, the third light emitting sources 280, 282 and 284 may provide at least one of ultraviolet light, visible light, and infrared light. For example, the third light emitting sources 280, 282 and 284 may be light emitting diodes (LEDs). The third light emitting sources 280, 282 and 284 may be provided in plural. The plurality of third light emitting sources 280, 282 and 284 may form one set. The third light emitting sources 280, 282 and 284 may have a triangular arrangement as one set. The third light emitting sources 280, 282 and 284 surround at least one of the image sensor 180 and the lens 170.

The third light emitting sources 280, 282 and 284 may provide red, green, and blue light. The third light emitting sources 280, 282 and 284 may provide light of at least one of an ultraviolet region, a visible light region, and a (near) infrared region. For example, the third light emitting sources 280, 282 and 284 may provide light with a wavelength of 405 nm in the ultraviolet region and light with wavelengths of 930 nm and 960 nm in the near-infrared region.

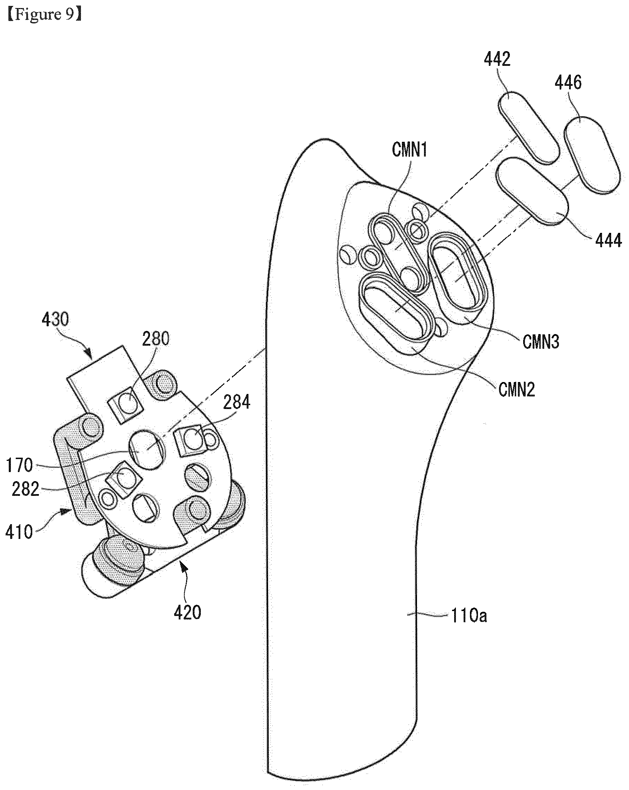

Referring to FIG. 9, a body inner portion 110a may form an inner portion of the body 110. The body inner portion 110a may accommodate the housing 410, the rear case 420, and the deck 430. The body inner portion 110a may include hollow pillars CMN1, CMN2 and CMN3.

The hollow pillars CMN1, CMN2 and CMN3 may have a shape protruding from a part of the body inner portion 110a toward the outside. The hollow pillars CMN1, CMN2 and CMN3 may pass light. For example, the hollow pillars CMN1, CMN2, CMN3 may be hollow. The hollow pillars CMN1, CMN2 and CMN3 may be provided in plural. For example, the hollow pillars CMN1, CMN2 and CMN3 may include a first hollow pillar CMN1, a second hollow pillar CMN2, and a third hollow pillar CMN3. The first hollow pillar CMN1 may be positioned corresponding to the third light emitting source 280 and the first opening OPN1. The second hollow pillar CMN2 may be positioned corresponding to the third light emitting source 282 and the second opening OPN2. The third hollow pillar CMN3 may be positioned corresponding to the third light emitting source 284 and the third opening OPN3.

Windows 442, 444 and 446 may be inserted into the hollow pillars CMN1, CMN2 and CMN3. The windows 442, 444 and 446 may pass light. The windows 442, 444 and 446 may pass at least a portion of light that has passed through the hollow pillars CMN1, CMN2 and CMN3. The windows 442, 444 and 446 may filter the passing light. For example, the windows 442, 444 and 446 may pass or block light of a specific region band. The windows 442, 444 and 446 may change or modify a path of the passing light. For example, windows 442, 444 and 446 may converge or disperse the passing light.

The windows 442, 444 and 446 may shield at least a portion of the hollow pillars CMN1, CMN2 and CMN3. The windows 442, 444 and 446 can prevent dust, etc. from entering into the hollow pillars CMN1, CMN2 and CMN3. The windows 442, 444 and 446 may be provided in plural. For example, the windows 442, 444 and 446 may include a first window 442, a second window 444, and a third window 446. The first window 442 may be connected to the first hollow pillar CMN1. The second window 444 may be connected to the second hollow pillar CMN2. The third window 446 may be connected to the third hollow pillar CMN3.

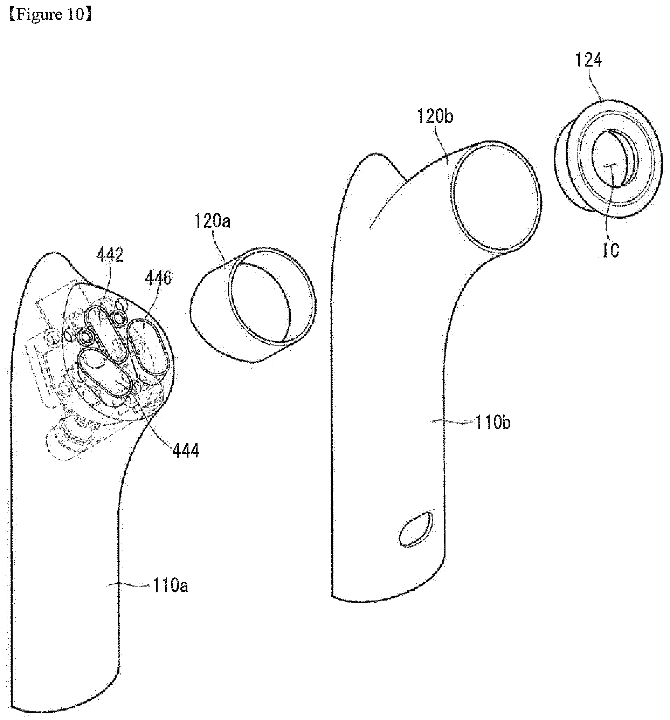

Referring to FIG. 10, a first head inner portion 120a may be connected to one side of the body inner portion 110a. The periphery of one end of the first head inner portion 120a may have a shape surrounding the hollow pillars CMN1, CMN2 and CMN3.

The first head inner portion 120a may pass light. For example, the first head inner portion 120a may form a hollow portion. A body outer portion 110b may form an outer portion of the body 110. The body outer portion 110b may face the body inner portion 110a. The first head outer portion 120b may form an outer portion of the first head 120. The first head outer portion 120b may be connected to a first head inner portion 120a. The first head outer portion 120b may have a shape extending from the body outer portion 110b in one direction. The first head outer portion 120b may be formed in one body with the body outer portion 110b.

The contact portion 124 may be positioned at an end of the first head outer portion 120b. The contact portion 124 may pass light. For example, the contact portion 124 may include a light transmission circle IC that allows light to pass through an inner periphery. The contact portion 124 may be directed toward or face an object. For example, the contact portion 124 may be directed toward or face the user's skin.

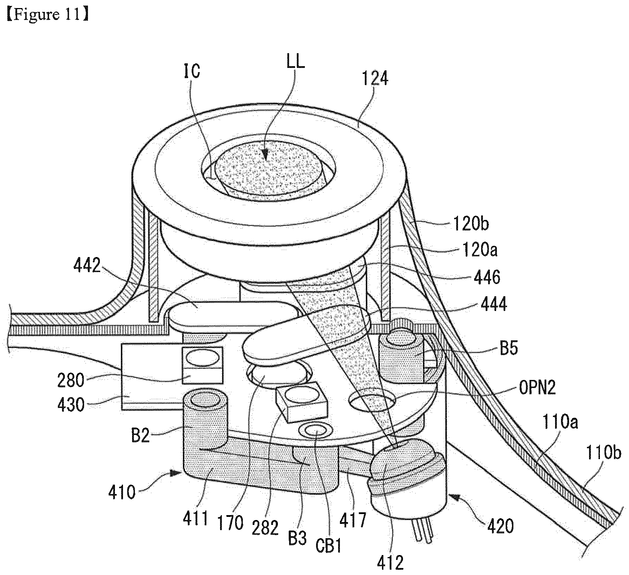

FIG. 11 illustrates a part of an electronic device according to an embodiment of the disclosure. With reference to FIG. 11, a flow of light related to speckle imaging will be described. The components required to describe the flow of light related to speckle imaging will refer to the components illustrated in FIGS. 7 to 10.

The first light emitting sources 260 and 262 shown in FIG. 7 may be laser diodes. The first light emitting sources 260 and 262 in FIG. 11 may be hidden by the housing holder 412 and the rear case 420 and may be invisible. The first light emitting sources 260 and 262 may be provided in plural and may provide light of different wavelengths. Light provided by the first light emitting sources 260 and 262 may be laser light LL as shown in FIG. 11. Light provided by the first light emitting sources 260 and 262 may pass through the housing holders 412 and 414. The light passing through the housing holders 412 and 414 may pass through the openings OPN2 and OPN3. The light passing through the openings OPN2 and OPN3 may pass through the windows 444 and 446 via the hollow pillars CMN2 and CMN3. The light passing through the windows 444 and 446 may reach the user's skin via the first head inner portion 120a. When the light provided by the first light emitting sources 260 and 262 reaches the user's skin, electromagnetic waves of different wavelengths may interfere with each other and/or may act on a skin tissue.

The third light emitting sources 280, 282 and 284 shown in FIG. 8 may provide light toward the light transmission circle IC. Light provided by the third light emitting sources 280, 282 and 284 may pass through the windows 442, 444 and 446 via the hollow pillars CMN1, CMN2 and CMN3 and reach the user's skin. The light provided by the third light emitting sources 280, 282 and 284 can improve or enhance an image obtained by the image sensor 180.

Light reflected from the user's skin may pass through the window 442 and reach the lens 170 via the hollow pillar CMN1. The lens 170 may change a path of light. For example, the lens 170 may converge or disperse light. Light passing through the lens 170 may reach the image sensor 180. The image sensor 180 may obtain an image. The image obtained by the image sensor 180 may include information about the skin condition of the user.

In the flow of light related to speckle imaging, a noise reduction of light may be considered. When a noise included in light reaching the user's skin is reduced, the quality of a speckle image can be improved. When a noise included in light that is provided from the user's skin and reaches the image sensor 180 is reduced, the quality of an image obtained by the image sensor 180 can be improved.

In order to reduce or suppress the noise of light, a light absorbing material may be included in an external surface of at least one of the housing 410, the rear case 420, and the deck 430. The light absorbing material may be included in the external surfaces of the third light emitting sources 280, 282 and 284. The light absorbing material may be included in an internal surface of the first head inner portion 120a. The light absorbing material may absorb light irradiated onto the component to which the light absorbing material is applied, thereby reducing the noise. The description of the light absorbing material may be equally applied to the above description.

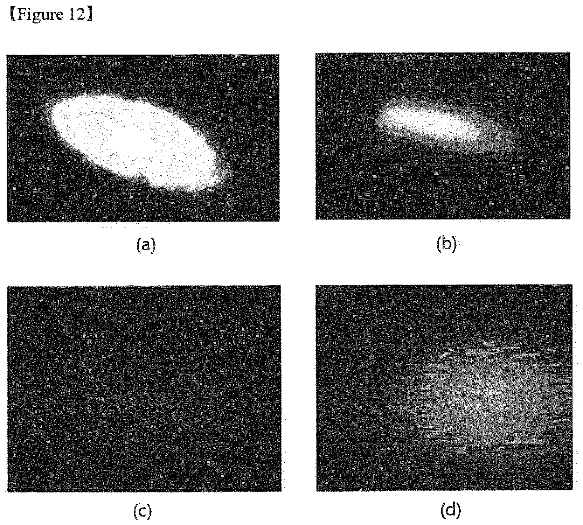

FIG. 12 illustrates examples of a measurement image according to an embodiment of the disclosure. FIG. 12(a) illustrates an example of a measured image of an object obtained by providing light with a wavelength of 405 nm, FIG. 12(b) illustrates an example of a measured image of an object obtained by providing light with a wavelength of 650 nm, FIG. 12(c) illustrates an example of a measured image of an object obtained by providing ultraviolet (UV) light, and FIG. 12(d) illustrates an example of a measured image of an object obtained by providing visible light. In the configurations described above, FIGS. 12(a) and 12(b) illustrate speckle imaging, and FIGS. 12(c) and 12(d) illustrate an image of an object in which diffuse reflection can be removed. This may be the effect obtained through the configuration of FIG. 11, or the effect obtained by other embodiments. More specifically, for example, information about the skin condition such as roughness, elasticity, oil, and moisture of the user's skin may be obtained by the images shown in FIGS. 12(a) and 12(b). Information about the skin condition such as oil, moisture, pores, pigment of the user's skin may be obtained by the images shown in FIGS. 12(c) and 12(d).

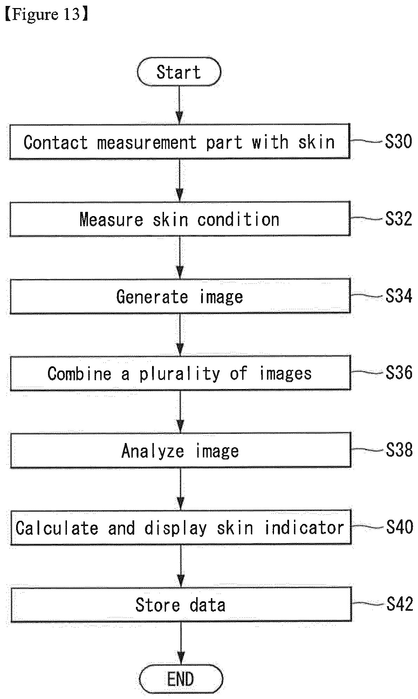

FIG. 13 illustrates an example of order of skin measurement according to an embodiment of the disclosure. In embodiments, the description of the same configuration according to the same reference numerals described above may be omitted. The user brings an electronic device into contact with his/her skin in S30. In this instance, the electronic device may detect whether it is in contact with the user's skin. When the user provides an input to an operation unit, the electronic device may operate. When the user provides an input to the operation unit and brings a measurement part into contact with his/her skin, the electronic device may operate at the same time as or after the contact of the measurement part and the user's skin. The electronic device may measure the skin condition of the user in S32. The measurement of the skin condition may generate an image by providing light to the skin by a first light emitting source and a third light emitting source and receiving light reflected from the skin by a lens and a sensor in S34.

A plurality of images may be generated. For example, an image may be generated at 30 to 50 frames per second by 405 nm light, an image may be generated at 30 to 50 frames per second by 650 nm light, and an image may be generated at 1 to 10 frames per second by light provided by ultraviolet LEDs, infrared LEDs, and visible LEDs. The images thus generated may be combined together to analyze the skin condition in S36. By the skin condition analysis in S38, skin indicators may be calculated in S40. Examples of the skin indicators may include roughness, elasticity, oil, moisture, pores, freckles, tone, and the like. The calculated skin indicators may be displayed through another terminal connected to the electronic device in S40. The calculated skin indicators may be stored for a history understanding of the user's skin condition which will be described later, and/or for skin care of the user in S42.

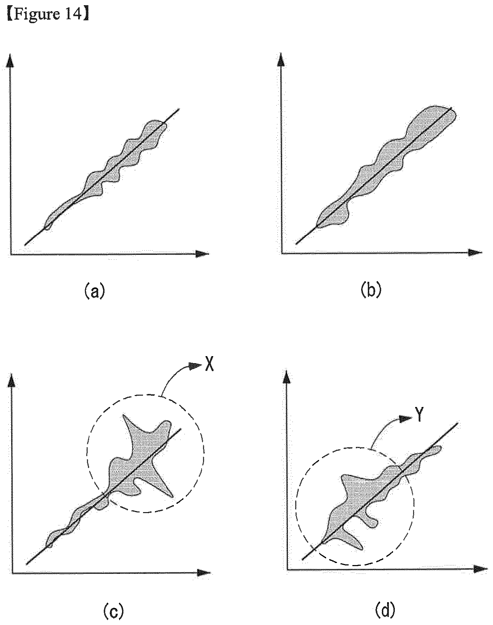

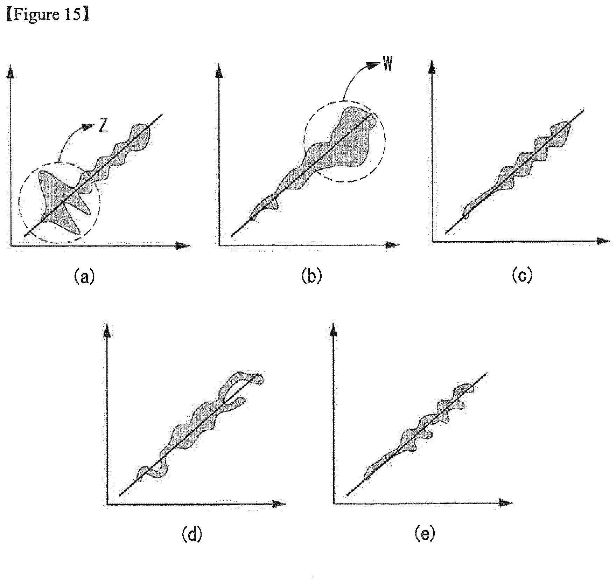

FIGS. 14 and 15 illustrate examples of a result of measuring skin conditions according to an embodiment of the disclosure. More specifically, FIG. 14 illustrates examples of a result of measuring skin conditions using a first light emitting source, and FIG. 15 illustrates examples of a result of measuring skin conditions using a third light emitting source.

FIG. 14(a) illustrates the distribution of a roughness state of a measured skin, FIG. 14(b) illustrates the distribution of an elasticity state of a measured skin, FIG. 14(c) illustrates the distribution of an oil state of a measured skin, and FIG. 14(d) illustrates the distribution of a moisture state of a measured skin. It should be noted that the distribution of an area X in FIG. 14(c) and the distribution of an area Y in FIG. 14(d) are dispersed. The dispersion may mean that the accuracy or the precision of the measurement is low.

FIG. 15(a) illustrates the distribution of an oil state of a measured skin, FIG. 15(b) illustrates the distribution of a moisture state of a measured skin, FIG. 15(c) illustrates the distribution of a pore state of a measured skin, FIG. 15(d) illustrates the distribution of a freckle state of a measured skin, and FIG. 15(e) illustrates the distribution of a tone state of a measured skin. It should be noted that the distribution of an area Z in FIG. 15(a) and the distribution of an area W in FIG. 15(b) are dispersed. The dispersion may mean that the accuracy or the precision of the measurement is low.

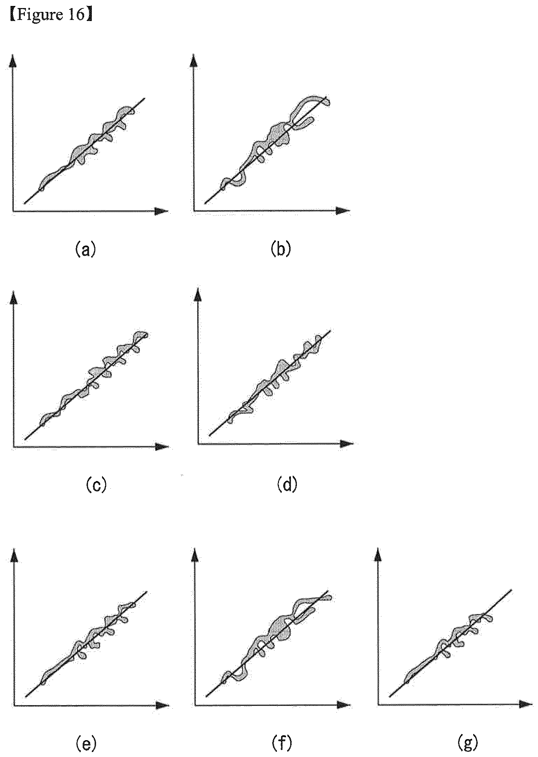

FIG. 16 illustrates other examples of a result of measuring skin conditions according to an embodiment of the disclosure.

FIG. 16(a) illustrates the distribution of a roughness state of a measured skin, FIG. 16(b) illustrates the distribution of an elasticity state of a measured skin, FIG. 16(c) illustrates the distribution of an oil state of a measured skin, and FIG. 16(d) illustrates the distribution of a moisture state of a measured skin. FIG. 16(e) illustrates the distribution of a pore state of a measured skin, FIG. 16(f) illustrates the distribution of a freckle state of a measured skin, and FIG. 16(g) illustrates the distribution of a tone state of a measured skin. It should be noted that the distributions of all the states, particularly, the oil state and the moisture state appear even. The even dispersion may mean that the accuracy or the precision of the measurement is high.

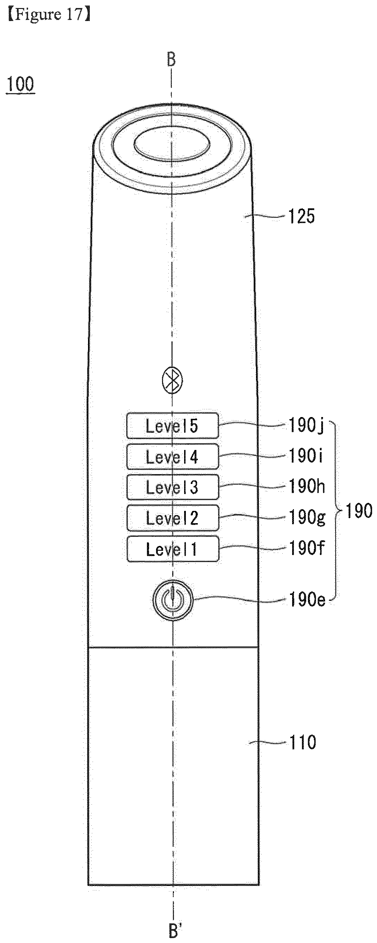

FIG. 17 is a figure of the electronic device of FIG. 1 viewed from a side of a second head. Referring to FIG. 17, the operation unit 190 may include a fifth operation unit 190e, a sixth operation unit 190f, a seventh operation unit 190g, an eighth operation unit 190h, a ninth operation unit 190i, and a tenth operation unit 190j.

The fifth operation unit 190e may obtain an input for power supply of at least some of the components included in the electronic device 100. For example, the fifth operation unit 190e may obtain an input for power supply of a component connected to the second head 125. For example, if the user presses the fifth operation unit 190e, skin measurement may be started.

The sixth to tenth operation units 190f, 190g, 190h, 190i and 190j may be related to the selection of functions of the electronic device 100. For example, the skin care corresponding to an input obtained by the sixth operation unit 190f may be different from the skin care corresponding to an input obtained by the tenth operation unit 190j.

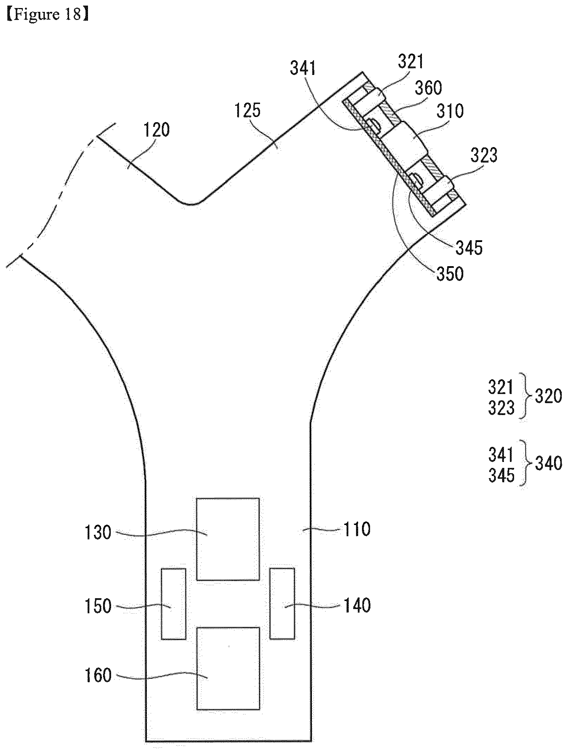

FIG. 18 is a cross-sectional view of an electronic device taken along B-B' of FIG. 17. Referring to FIG. 18, a first head 120 may be extended from a body 110 in one direction. A second head 125 may be extended from the body 110 in another direction. The configuration disposed at the first head 120 may be omitted in FIG. 18. The description of a main circuit board 130, a light control unit 140, a wireless communication unit 150, and a power supply unit 160, etc. may include the above description.

A first electrode 310 and a second electrode 320 may be positioned on an external surface of the second head 125. At least a portion of the first electrode 310 and at least a portion of the second electrode 320 may be exposed to the outside. The first electrode 310 and the second electrode 320 may have an electric potential. For example, the first electrode 310 may have a first electric potential, and the second electrode 320 may have a second electric potential. The second electrode 320 may be provided in plural. For example, the second electrode 320 may include a plurality of second electrodes 321 and 323. The first electrode 310 and the second electrode 320 may be connected to a second circuit board 350. The first electrode 310 and the second electrode 320 may receive power and/or electrical signals from the second circuit board 350.

A skin contact portion 360 may be positioned on the external surface of the second head 125. The skin contact portion 360 may accommodate the first electrode 310 and the second electrode 320. The skin contact portion 360 may be in contact with the user's skin. The skin contact portion 360 may have electrical insulation.

A second light emitting source 340 may be connected to the second circuit board 350. The second light emitting source 340 may receive power and/or electrical signals from the second circuit board 350. The second light emitting source 340 may be disposed between the first electrode 310 and the second electrode 320.

The second light emitting source 340 may provide light. For example, the second light emitting source 340 may be an LED. The second light emitting source 340 may provide light energy. The second light emitting source 340 may provide heat energy. The second light emitting source 340 may simultaneously provide both light energy and thermal energy. The second light emitting source 340 may be provided in plural. For example, the second light emitting source 340 may include a plurality of second light emitting sources 341 and 345.

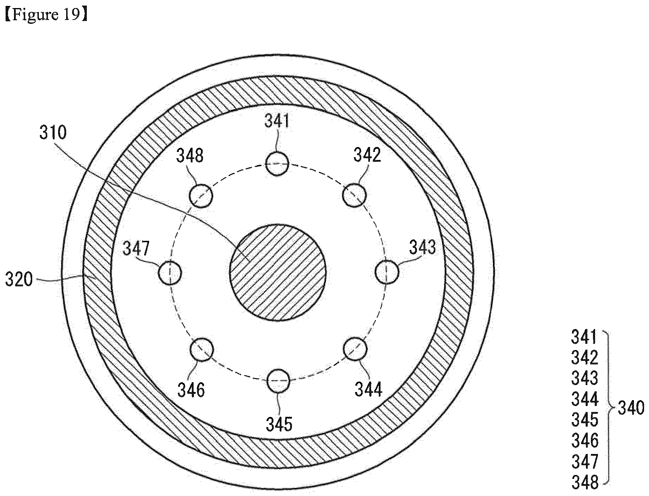

FIG. 19 illustrates an example of a front surface of a second head according to an embodiment of the disclosure. Referring to FIG. 19, a second electrode 320 may have a shape surrounding a first electrode 310. The second electrode 320 may have a ring shape. The second electrode 320 may be formed in one body. The second electrode 320 may be spaced from the first electrode 310.

The second light emitting source 340 may be disposed between the first electrode 310 and the second electrode 320. The second light emitting source 340 may be spaced from the first electrode 310. The second light emitting source 340 may be spaced from the second electrode 320. The second light emitting source 340 may include a plurality of second light emitting sources 341, 342, 343, 344, 345, 346, 347 and 348.

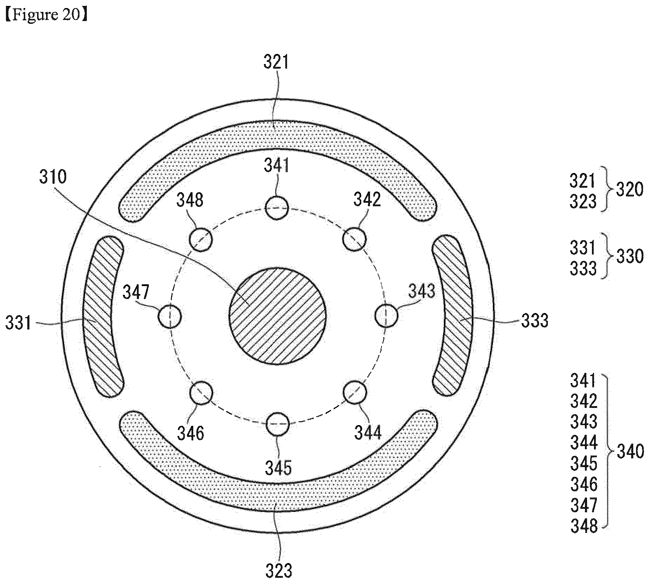

FIG. 20 illustrates an example of a front surface of a second head according to an embodiment of the disclosure. Referring to FIG. 20, a second electrode 320 may be provided in plural. For example, the second electrode 320 may include a plurality of second electrodes 321 and 323. The first electrode 310 may be disposed between the second electrodes 321 and 323.

A third electrode 320 may be provided in plural. For example, the third electrode 330 may include a plurality of third electrodes 331 and 333. The first electrode 310 may be disposed between the third electrodes 331 and 333. The third electrode 320 may have an electric potential. For example, the third electrode 320 may have a third electric potential.

The second light emitting source 340 may be disposed between the first electrode 310 and the second electrode 320. The second light emitting source 340 may be disposed between the first electrode 310 and the third electrode 330.

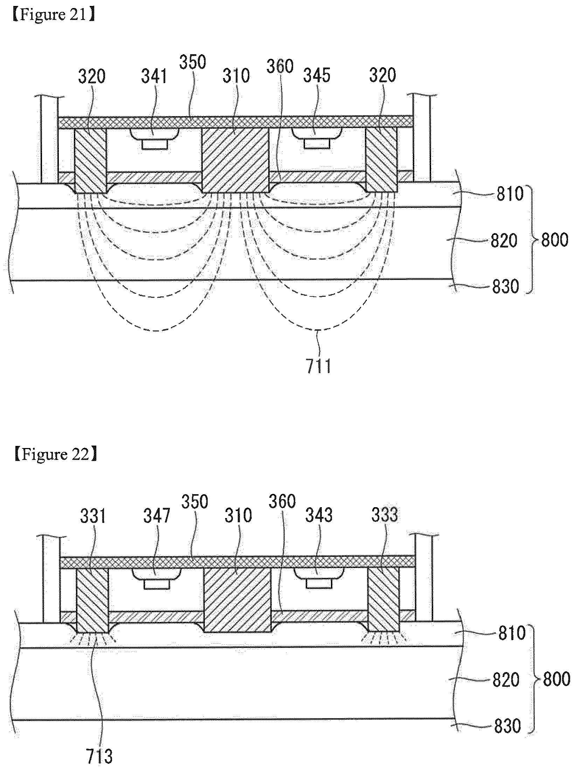

FIGS. 21 to 23 illustrate implementations of a skin care performed by an electronic device according to an embodiment of the disclosure.

Referring to FIG. 21, the first electrode 310 and the second electrode 320 may contact a skin 800. The first electrode 310 and the second electrode 320 may be connected to an AC power source. For example, the AC power connected to the first electrode 310 and the second electrode 320 may have a frequency of a radio frequency (RF) region. The first electrode 310 may have a first electric potential. The second electrode 320 may have a second electric potential. The first electric potential and the second electric potential may have the same magnitude and different phases.

The first electrode 310 and the second electrode 320 may contact the skin 800 and provide energy to the skin 800. When the first electrode 310 and the second electrode 320 are connected to the AC power source and contact the skin 800, an electromagnetic wave 711 may proceed to the skin 800. The electromagnetic wave 711 passing through the skin 800 may be an RF wave. The electromagnetic wave 711 may involve heat.

The skin 800 may include an epidermis 810, a dermis 820, and a subcutaneous fat 830. The epidermis 810, the dermis 820, and the subcutaneous fat 830 may be sequentially positioned. The epidermis 810 may contact the first electrode 310 and the second electrode 320. The electromagnetic wave 711 provided by the first electrode 310 and the second electrode 320 may provide energy to at least one of the epidermis 810, the dermis 820, and the subcutaneous fat 830.

FIG. 22 illustrates that the third electrode 330 acts on the skin. Referring to FIG. 22, the third electrode 330 may include third electrodes 331 and 333. The third electrode 330 may contact the skin 800. The third electrode 330 may be connected to an AC power source or a DC power source. The third electrode 330 may have a third electric potential. The third electrode 330 may provide thermal energy to the skin 800. The manner in which the third electrode 330 provides thermal energy to the skin 800 may be at least one of radiation and conduction. For example, an electromagnetic wave 713 generated by the third electrode 330 may provide thermal energy to the skin 800. For example, the electromagnetic wave 713 provided by the third electrode 330 may be an infrared wave.

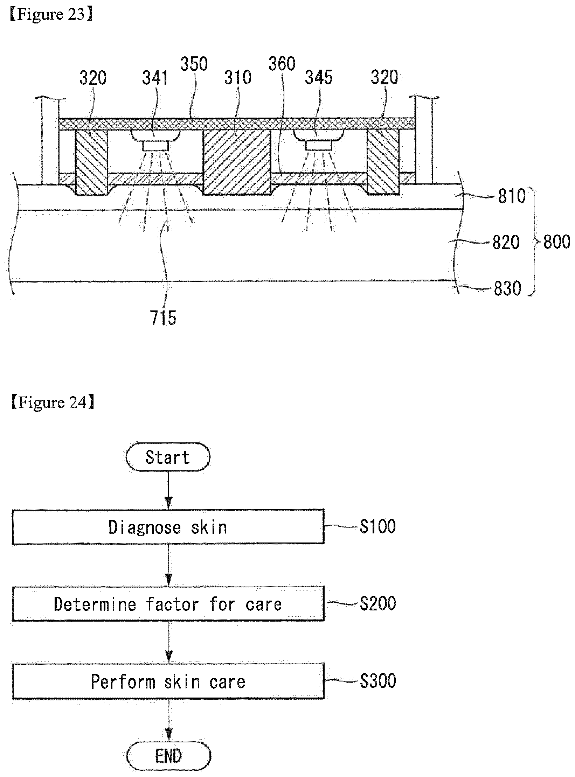

FIG. 23 illustrates that the second light emitting source 340 acts on the skin. Referring to FIG. 23, the second light emitting source 340 may be spaced from the skin 800. The second light emitting source 340 may be provided in plural. For example, the second light emitting source 340 may include second light emitting sources 341 and 345. The second light emitting source 340 may provide light to the skin 800. The second light emitting source 340 may provide thermal energy to the skin 800. The manner in which the second light emitting source 340 provides energy to the skin 800 may be radiation. For example, an electromagnetic wave 715 provided by the second light emitting source 340 may be a visible light wave.

FIG. 24 illustrates order of skin measurement and skin care according to an embodiment of the disclosure. The electronic device according to an embodiment of the disclosure can measure the skin and can care for the skin. In embodiments, order of skin measurement and skin care may be considered. In embodiments, the description of the same configuration according to the same reference numerals described above may be omitted.

The electronic device may contact a user's skin and diagnose the skin in S100. A process for diagnosing the user's skin by the electronic device may include a process for measuring the user's skin and analyzing an image by the electronic device. The electronic device may obtain information on a skin condition of the user.

The electronic device may determine a factor for care according to the user's skin condition in S200. The care factor may correspond to the user's skin condition. For example, when the user's skin is deficient in water, the care factor may be determined to promote the formation of an oil film on the skin. The care factor may mean an input value for driving at least one of the first electrode, the second electrode, the third electrode, and the second light emitting source included in the electronic device. For example, a controller of the electronic device may determine a care factor for driving at least one of the first electrode, the second electrode, the third electrode, and the second light emitting source according to the user's skin condition.

The electronic device may provide the skin care to the user according to the determined care factor in S300. For example, the controller of the electronic device may drive at least one of the first electrode, the second electrode, the third electrode, and the second light emitting source according to the determined care factor.

The electronic device can be easy for the user to carry. The electronic device can continuously provide diagnosis and care of the skin to the user. The user may cause the electronic device to diagnose the skin after performing the skin care in S100.

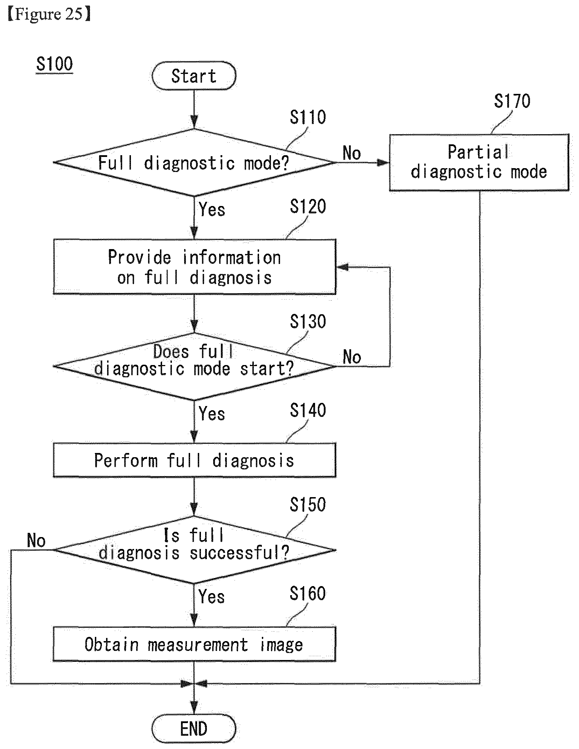

FIG. 25 illustrates order of skin diagnosis according to an embodiment of the disclosure. The electronic device may communicate with an external terminal. The manner in which the electronic device communicates with the external terminal may be wired and/or wireless manner. The electronic device may operate through an application installed in the external terminal.

The electronic device may have two modes for skin diagnosis. For example, the electronic device may have a full diagnostic mode and a partial diagnostic mode. The full diagnostic mode may be a mode for diagnosis of a full face. The partial diagnostic mode may be a mode for diagnosis of a part of a face. The electronic device may determine whether it is in the full diagnostic mode in S110.

In case of the full diagnostic mode, the electronic device may provide information about the full diagnosis to the external terminal in S120. The user may recognize that the electronic device is in the full diagnostic mode. If the user operates the electronic device in the full diagnostic mode in S130, the electronic device may perform the full diagnosis on the user's skin in the full diagnostic mode in S140. If the user has not operated the electronic device in the full diagnostic mode in S130, the electronic device may continue to provide information about the full diagnosis to the external terminal in S120.

The electronic device may determine whether the full diagnosis performed is successful in S150. If the full diagnosis performed is successful, the electronic device may obtain a measurement image in S160.

If the electronic device is in the partial diagnostic mode, the electronic device may diagnose the user's skin in the partial diagnostic mode in S170.

The electronic device can diagnose the user's skin without being linked to the external terminal. The electronic device may determine whether it is in the full diagnostic mode in S110. If the user operates the electronic device in the full diagnostic mode, the electronic device may perform the full diagnosis on the user's skin in the full diagnostic mode in S140. The electronic device may determine whether the full diagnosis performed is successful in S150. If the full diagnosis performed is successful, the electronic device may obtain a measurement image in S160. If the electronic device is in the partial diagnostic mode, the electronic device may diagnose the user's skin in the partial diagnostic mode in S170.

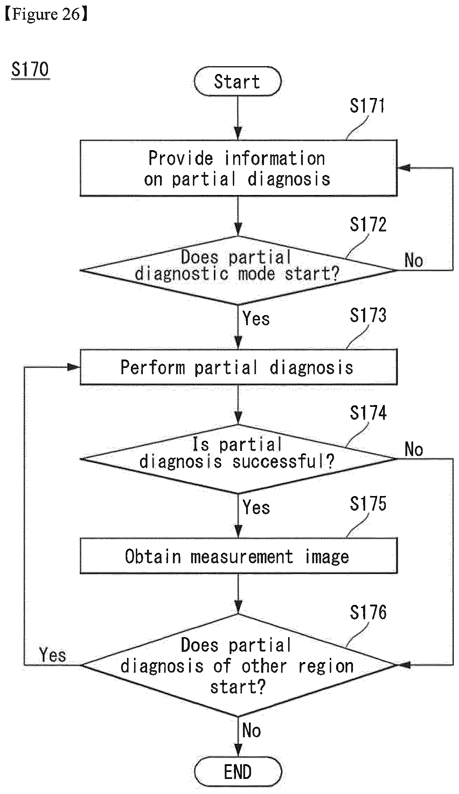

FIG. 26 illustrates order of execution of a partial diagnostic mode according to an embodiment of the disclosure.

In case of the partial diagnostic mode, the electronic device may provide information about a partial diagnosis to the external terminal in S171. The user may recognize that the electronic device is in the partial diagnostic mode. If the user operates the electronic device in the partial diagnostic mode in S172, the electronic device may perform the partial diagnosis on the user's skin in the partial diagnostic mode in S173. If the user has not operated the electronic device in the partial diagnostic mode in S172, the electronic device may continue to provide information about the partial diagnosis to the external terminal in S171.

The electronic device can diagnose the user's skin without being linked to the external terminal. If the user operates the electronic device in the partial diagnostic mode, the electronic device may perform the partial diagnosis on the user's skin in the partial diagnostic mode in S173.

The electronic device may determine whether the partial diagnosis performed is successful in S174. If the partial diagnosis performed is successful, the electronic device may obtain a measurement image in S175. If the partial diagnosis performed is not successful, the electronic device may receive an input for starting a partial diagnosis of other region from the user. When the electronic device obtains the measurement image, the electronic device may determine whether to receive an input for starting a partial diagnosis of other region from the user in S176.

If the electronic device receives an input for starting a partial diagnosis of other region from the user, the electronic device may perform the partial diagnosis on the other region in S173. If the electronic device does not receive an input for starting a partial diagnosis of other region from the user, the electronic device may end the partial diagnostic mode.

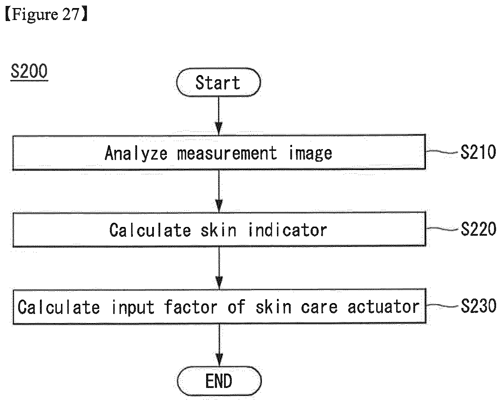

FIG. 27 illustrates order of determination of a care factor in accordance with an embodiment of the disclosure.

The electronic device may analyze a measured image for the user's skin in S210. The electronic device may calculate a skin indicator corresponding to the measured image. The measured image may correspond to the calculated skin indicator. For example, the measured image may correspond to the skin indicator via a neural network.

The calculated skin indicator may include information about the skin condition such as roughness, elasticity, oil, and moisture of the user's skin. The electronic device may calculate an input factor of a skin care actuator correspondingly to the calculated skin indicator in S230. The skin care actuator may provide energy, etc. to the user's skin and may change the user's skin. For example, the skin care actuator may include at least one of the first electrode, the second electrode, the third electrode, and the second light emitting source according to an embodiment of the disclosure.

The input factor of the skin care actuator may include, for example, selection of a suitable skin care actuator among a plurality of skin care actuators to improve the skin. For example, the input factor of the skin care actuator may include an intensity of an electrical signal provided to the skin care actuator and/or a magnitude of power, and the like. In embodiments, the magnitude of power may include a magnitude of a voltage and/or a magnitude of a current.

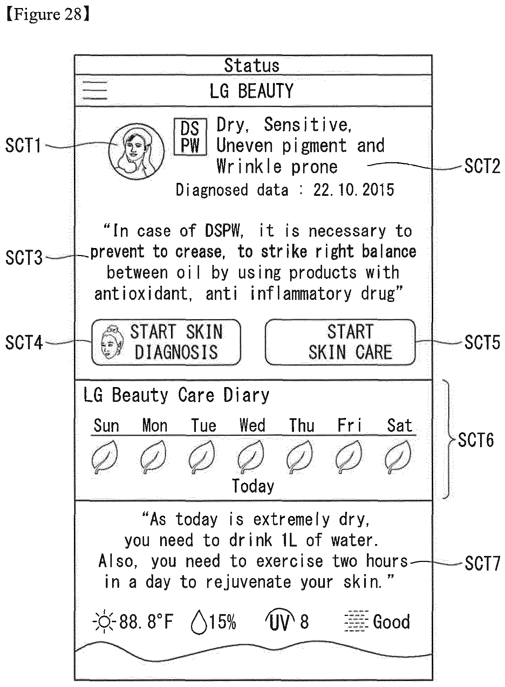

FIGS. 28 to 30 illustrate a terminal connected to an electronic device according to an embodiment of the disclosure. FIGS. 28 to 30 illustrate a screen displayed on a display unit of an external terminal. In embodiments, the external terminal may be connected to the electronic device according to an embodiment of the disclosure.

FIG. 28 illustrates a screen displayed on an external terminal in accordance with an embodiment of the disclosure. FIG. 28 illustrates first to seventh sectors SCT1 to SCT7.

The first sector SCT1 may display an ID of the user. For example, the first sector SCT1 may display a user's face on the screen.

The second sector SCT2 may display information about a recent skin measurement of the user. The second sector SCT2 may display time at which the user measures his/her skin.

The third sector SCT3 may display information according to a diagnosis result displayed on the second sector SCT2. For example, the third sector SCT3 may display information about a skin care in response to the diagnosis result.

The fourth sector SCT4 may receive an input for performing a skin diagnosis. If the user provides the terminal with the input for performing the skin diagnosis, the electronic device may perform the skin diagnosis.

The fifth sector SCT5 may receive an input for performing the skin care. If the user provides the terminal with the input for performing the skin care, the electronic device may perform the skin care.

The sixth sector SCT6 may display a history of skin diagnosis and/or skin care. For example, the sixth sector SCT6 may display how many days ago the skin was diagnosed and/or cared.

The seventh sector SCT7 may display current weather or climate conditions. The seventh sector SCT7 may display a skin care tip suitable for the current weather or climate.

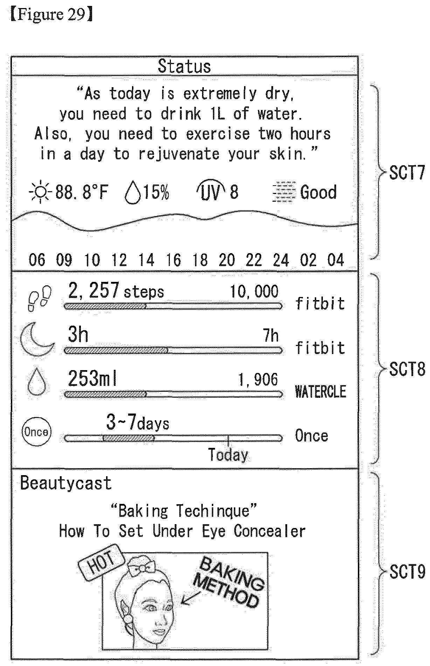

FIG. 29 illustrates a screen displayed on an external terminal in accordance with an embodiment of the disclosure. FIG. 29 illustrates seventh to ninth sectors SCT7 to SCT9.

The seventh sector SCT7 may display a skin care tip together with current weather or climate conditions.

The eighth sector SCT8 may display information about a user's activity. For example, the eighth sector SCT8 may display how long the user has walked. For example, the eighth sector SCT8 may display a water intake or sleeping time of the user, etc. The information about the user's activity may be reflected in the skin condition or the skin care of the user.

The ninth sector SCT9 may display advertisements for skin beauty. Or the ninth sector SCT9 may display podcasts about skin beauty.

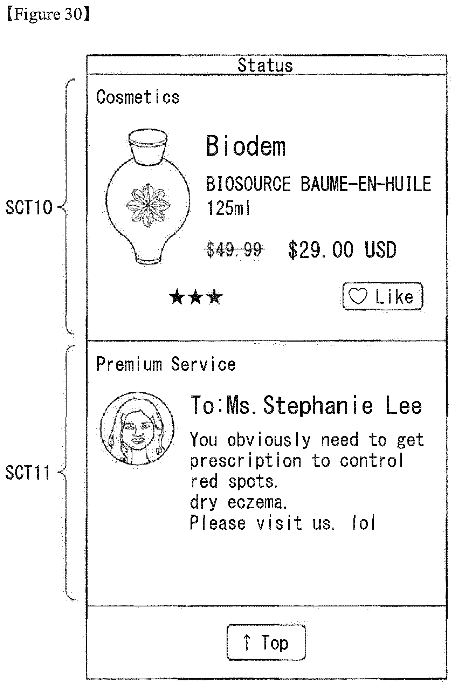

FIG. 30 illustrates a screen displayed on an external terminal in accordance with an embodiment of the disclosure. FIG. 30 illustrates tenth and eleventh sectors SCT10 and SCT11.

The tenth sector SCT10 may display a beauty product suitable for the measured skin condition of the user. The tenth sector SCT10 may display an appearance, price, quantity, etc. of the beauty product.

The eleventh sector SCT11 may display expert advice, etc. on the measured skin condition of the user.

Some embodiments of the disclosure described above or other embodiments are not mutually exclusive or distinct from each other. Some embodiments of the disclosure described above or other embodiments may be combined with each other in configuration or function.

It is obvious to those skilled in the art that the present disclosure can be employed in other specific forms within the scope of the appended claims.

## Claims

1. An electronic device (100) comprising:
a body (110) having an accommodation space therein;
a first head (120) extended from the body (110) in one direction and having an accommodation space therein;
a light transmission circle (IC) formed on a part of the first head (120);
a plurality of first light emitting sources (260, 262) having an irradiation axis, and being mounted on the accommodation space of at least one of the body (110) and the first head (120) and providing light for speckle imaging to the light transmission circle (IC);
an image sensor (180) and a lens (170) having an optical axis, wherein the image sensor (180) and the lens (170) are positioned inward the device (100) along the optical axis compared to the light transmission circle (IC);
a second head (125) extended from the body (110) in another direction;
a first electrode (310) and a second electrode (320) positioned on an external surface of the second head (125);
a second light emitting source (340) positioned adjacent to the first electrode (310) or the second electrode (320) and providing light to an outside of the second head (125); and
a third light emitting source (280, 282, 284) positioned around the lens (170) and providing light for taking an image, **characterized in that** the plurality of first light emitting sources (260, 262) are positioned inward of the device along the optical axis relative to the third light emitting source (280, 282, 284), and **in that** an angle (θ1, θ2) between the irradiation axis and the optical axis is 30 to 40 degrees.

2. The electronic device of claim 1, further comprising:
a controller configured to analyze an image obtained by the image sensor (180) and calculate an indicator.

3. The electronic device of claim 2, wherein the controller is configured to drive at least one of the first electrode (310), the second electrode (320), and the second light emitting source (340) according to the calculated indicator.

4. The electronic device of claim 1, wherein the plurality of first light emitting sources (260, 262) is laser diodes.

5. The electronic device of claim 1, further comprising a housing (410) including:
a plurality of housing holders (412, 414) accommodating the plurality of first light emitting sources (206, 262);
a housing body (411) including a housing accommodation portion (415) accommodating the image sensor (180), the housing body (411) being positioned between the light transmission circle (IC) and the plurality of housing holders (412, 414); and
a plurality of housing legs (417, 418) connecting the housing body (411) to the plurality of housing holders (412, 414).

6. The electronic device of claim 5, further comprising:
a rear case (420) coupled to a rear of the housing (410) and contacting the plurality of first light emitting sources (260, 262),
wherein the rear case (420) includes a metal.

7. The electronic device of claim 6, wherein at least one of the housing (410) and the rear case (420) includes a light absorbing material that is a material capable of absorbing light in at least one of visible light, infrared light, and ultraviolet light on an external surface.

8. The electronic device of claim 1, further comprising a third electrode (330) positioned adjacent to at least one of the first electrode (310) and the second electrode (320) and providing light of an infrared region,
wherein the first electrode (310) and the second electrode (320) each have an electric potential,
wherein the first electrode (310) has a first electric potential, and the second electrode (320) has a second electric potential different from the first electric potential.

## Patentansprüche

1. Elektronische Vorrichtung (100), umfassend:
einen Körper (110) mit einem Aufnahmeraum darin;
einen ersten Kopf (120), der sich von dem Körper (110) in eine Richtung erstreckt und einen Aufnahmeraum darin aufweist;
einen Lichtdurchlasskreis (IC), der auf einem Teil des ersten Kopfes (120) ausgebildet ist;
eine Mehrzahl von ersten Lichtquellen (260, 262), die eine Ausstrahlachse haben und in dem Aufnahmeraum des Körpers (110) und/oder des ersten Kopfes (120) angebracht sind und dem Lichtdurchlasskreis (IC) Licht zur Speckle-Abbildung zuführen;
einen Bildsensor (180) und eine Linse (170) mit einer optischen Achse, wobei der Bildsensor (180) und die Linse (170) im Inneren der Vorrichtung (100) entlang der optischen Achse im Vergleich zum Lichtdurchlasskreis (IC) angeordnet sind;
einen zweiten Kopf (125), der sich vom Körper (110) in eine andere Richtung erstreckt;
eine erste Elektrode (310) und eine zweite Elektrode (320), die auf einer Außenfläche des zweiten Kopfes (125) angeordnet sind;
eine zweite Lichtquelle (340), die neben der ersten Elektrode (310) oder der zweiten Elektrode (320) angeordnet ist und Licht an die Außenseite des zweiten Kopfes (125) liefert; und
eine dritte Lichtquelle (280, 282, 284), die um die Linse (170) herum angeordnet ist und Licht zur Aufnahme eines Bildes liefert,
**dadurch gekennzeichnet, dass** die Mehrzahl von ersten Lichtquellen (260, 262) entlang der optischen Achse relativ zu der dritten Lichtquelle (280, 282, 284) innerhalb der Vorrichtung angeordnet sind, und
dass ein Winkel (θ1, θ2) zwischen der Ausstrahlachse und der optischen Achse 30 bis 40 Grad beträgt.

2. Elektronische Vorrichtung gemäß Anspruch 1, ferner umfassend:
eine Steuerung, die dazu eingerichtet ist, ein von dem Bildsensor (180) erhaltenes Bild zu analysieren und einen Indikator zu berechnen.

3. Elektronische Vorrichtung gemäß Anspruch 2, wobei die Steuerung dazu eingerichtet ist, mindestens eine der ersten Elektrode (310), der zweiten Elektrode (320) und der zweiten Lichtquelle (340) entsprechend dem berechneten Indikator anzusteuern.

4. Elektronische Vorrichtung gemäß Anspruch 1, wobei es sich bei der Mehrzahl von ersten Lichtquellen (260, 262) um Laserdioden handelt.

5. Elektronische Vorrichtung gemäß Anspruch 1, ferner ein Gehäuse (410) umfassend, das Folgendes enthält:
eine Mehrzahl von Gehäusehaltern (412, 414), die die Mehrzahl von ersten Lichtquellen (206, 262) aufnehmen;
einen Gehäusekörper (411) mit einem Gehäuseaufnahmeabschnitt (415), der den Bildsensor (180) aufnimmt, wobei der Gehäusekörper (411) zwischen dem Lichtdurchlasskreis (IC) und der Mehrzahl von Gehäusehaltern (412, 414) angeordnet ist; und
eine Mehrzahl von Gehäuseschenkeln (417, 418), die den Gehäusekörper (411) mit der Mehrzahl von Gehäusehaltern (412, 414) verbinden.

6. Elektronische Vorrichtung gemäß Anspruch 5, ferner umfassend:
ein hinteres Gehäuse (420), das mit einer Rückseite des Gehäuses (410) gekoppelt ist und die Mehrzahl von ersten Lichtquellen (260, 262) berührt,
wobei das hintere Gehäuse (420) aus einem Metall besteht.

7. Elektronische Vorrichtung gemäß Anspruch 6, wobei das Gehäuse (410) und/oder hintere Gehäuse (420) ein lichtabsorbierendes Material enthält, das ein Material ist, das in der Lage ist, Licht in mindestens einem der Bereiche sichtbares Licht, Infrarotlicht und ultraviolettes Licht auf einer äußeren Oberfläche zu absorbieren.

8. Elektronische Vorrichtung gemäß Anspruch 1, ferner eine dritte Elektrode (330) umfassend, die neben der ersten Elektrode (310) und/oder der zweiten Elektrode (320) angeordnet ist und Licht aus einem Infrarotbereich liefert,
wobei die erste Elektrode (310) und die zweite Elektrode (320) jeweils ein elektrisches Potential aufweisen,
wobei die erste Elektrode (310) ein erstes elektrisches Potential aufweist und die zweite Elektrode (320) ein zweites elektrisches Potential aufweist, das sich von dem ersten elektrischen Potential unterscheidet.

## Revendications

1. Dispositif électronique (100) comprenant:
un corps (110) comportant un espace de logement à l'intérieur;
une première tête (120) étendue depuis le corps (110) dans une direction et comportant un espace de logement à l'intérieur;
un cercle de transmission de lumière (IC) formé sur une partie de la première tête (120);
une pluralité de premières sources émettrices de lumière (260, 262), ayant un axe d'irradiation, et étant montées sur l'espace de logement d'au moins l'un du corps (110) et de la première tête (120) et fournissant de la lumière pour une imagerie par granularité au cercle de transmission de lumière (IC);
un capteur d'image (180) et une lentille (170) ayant un axe optique, dans lequel le capteur d'image (180) et la lentille (170) sont positionnés vers l'intérieur du dispositif (100) le long de l'axe optique par rapport au cercle de transmission de lumière (IC);
une seconde tête (125) étendue depuis le corps (110) dans une autre direction;
une première électrode (310) et une deuxième électrode (320) positionnées sur une surface externe de la seconde tête (125);
une deuxième source émettrice de lumière (340) positionnée adjacente à la première électrode (310) ou à la deuxième électrode (320) et fournissant de la lumière à l'extérieur de la seconde tête (125); et
une troisième source émettrice de lumière (280, 282, 284) positionnée autour de la lentille (170) et fournissant de la lumière pour prendre une image,
**caractérisé en ce que** la pluralité de premières sources émettrices de lumière (260, 262) sont positionnées vers l'intérieur du dispositif le long de l'axe optique par rapport à la troisième source émettrice de lumière (280, 282, 284), et **en ce qu'**un angle (θ1, θ2) entre l'axe d'irradiation et l'axe optique est de 30 à 40 degrés.

2. Dispositif électronique selon la revendication 1, comprenant en outre:
un contrôleur configuré pour analyser une image obtenue par le capteur d'image (180) et calculer un indicateur.

3. Dispositif électronique selon la revendication 2, dans lequel le contrôleur est configuré pour piloter au moins l'une parmi la première électrode (310), la deuxième électrode (320) et la deuxième source émettrice de lumière (340) en fonction de l'indicateur calculé.

4. Dispositif électronique selon la revendication 1, dans lequel la pluralité de premières sources émettrices de lumière (260, 262) sont des diodes laser.

5. Dispositif électronique selon la revendication 1, comprenant en outre un boîtier (410) comprenant:
une pluralité de supports de boîtier (412, 414) logeant la pluralité de premières sources émettrices de lumière (206, 262);
un corps de boîtier (411) comprenant une partie logement de boîtier (415) logeant le capteur d'image (180), le corps de boîtier (411) étant positionné entre le cercle de transmission de lumière (IC) et la pluralité de supports de boîtier (412, 414); et
une pluralité de pattes de boîtier (417, 418) reliant le corps de boîtier (411) à la pluralité de supports de boîtier (412, 414).

6. Dispositif électronique selon la revendication 5, comprenant en outre:
un châssis arrière (420) accouplé à un arrière du boîtier (410) et en contact avec la pluralité de premières sources émettrices de lumière (260, 262),
dans lequel le châssis arrière (420) comprend un métal.

7. Dispositif électronique selon la revendication 6, dans lequel au moins l'un du boîtier (410) et du châssis arrière (420) comprend un matériau absorbant la lumière qui est un matériau capable d'absorber la lumière dans au moins l'une parmi la lumière visible, la lumière infrarouge et la lumière ultraviolette sur une surface externe.

8. Dispositif électronique selon la revendication 1, comprenant en outre une troisième électrode (330) positionnée adjacente à au moins l'une de la première électrode (310) et de la deuxième électrode (320) et fournissant une lumière d'une région infrarouge,
dans lequel la première électrode (310) et la deuxième électrode (320) ont chacune un potentiel électrique,
dans lequel la première électrode (310) a un premier potentiel électrique, et la deuxième électrode (320) a un deuxième potentiel électrique différent du premier potentiel électrique.
